# EUROPÄISCHE PATENTSCHRIFT

(11) **EP 1 448 264 B1**
(45) Veröffentlichungstag und Bekanntmachung des Hinweises auf die Patenterteilung: **09.05.2007**
(21) Anmeldenummer: 02801300.1
(22) Anmeldetag: 01.10.2002
(51) Int. Cl.: A61N 1/36, A61N 1/08

(54) **RETINA-IMPLANTAT MIT ELEKTRODENANORDNUNG ZUR ELEKTRISCHEN STIMULATION VON BIOLOGISCHEM MATERIAL**
RETINA IMPLANT COMPRISING AN ELECTRODE ARRANGEMENT FOR ELECTRICALLY STIMULATING BIOLOGICAL MATERIAL
IMPLANT RETINIEN A AGENCEMENT D'ELECTRODES POUR LA STIMULATION ELECTRIQUE D'UNE MATIERE BIOLOGIQUE

(30) Priorität: 17.10.2001 DE 10151650
(43) Veröffentlichungstag der Anmeldung: 25.08.2004
(73) Patentinhaber: Retina Implant GmbH, 72770 Reutlingen (DE)
(72) Erfinder: STETT, Alfred, 72770 Reutlingen (DE); NISCH, Wilfried, 72070 Tübingen (DE); STELZLE, Martin, 72776 Reutlingen (DE); ZRENNER, Eberhart, Prof. Dr., 72076 Tübingen (DE)
(74) Vertreter: Otten, Hajo
(86) Internationale Anmeldenummer: PCT/EP2002/010972
(87) Internationale Veröffentlichungsnummer: WO 2003/033069

(56) Entgegenhaltungen:
- US-A- 4 310 000
- US-A- 5 674 264
- US-A- 5 944 747
- US-B1- 6 230 057
- US-B1- 6 269 268
- US-B1- 6 301 505

## Beschreibung

Die vorliegende Erfindung betrifft ein Retina-Implantat mit einer Elektrodenanordnung zur elektrischen Stimulation von biologischem Material, wobei die Elektrodenanordnung mit zumindest einer Stimulationselektrode, über die dem biologischen Material ein Stimulussignal zuführbar ist, und mit zumindest einer Gegenelektrode versehen ist, die einen Gegenpol zu der Stimulationselektrode bildet.

Ein derartiges Retina-Implantat ist aus der US 4,628,933 bekannt.

In dieser Druckschrift ist ein sogenanntes Retina-Implantat beschrieben, also ein Implantat, das im Bereich der Netzhaut (Retina) eines Auges eingesetzt werden soll. Das Retina-Implantat besitzt eine Vielzahl feldartig angeordneter Stimulationselektroden, mit denen künstlich erzeugte Stimulussignale bestimmten, in der Retina befindlichen Körperzellen zugeführt werden. Die Stimulussignale werden mit Hilfe lichtempfindlicher Elemente erzeugt, von denen das Retina-Implantat ebenfalls eine Vielzahl aufweist. Das beschriebene Implantat soll dazu dienen, Menschen, die bspw. infolge einer als Retinitis Pigmentosa bekannten Krankheit ihr Sehvermögen verloren haben, zumindest ein gewisses Sehvermögen wiederzubringen.

Die Stimulationselektroden des beschriebenen Retina-Implantats stehen nach der Implantation in direktem Kontakt mit umgebendem Zellgewebe und mit Körperflüssigkeiten, die im Bereich des Zellgewebes vorhanden sind. Es ist bekannt, daß sich an der Grenzschicht zwischen den Elektroden und dem Zellgewebe bzw. der Körperflüssigkeit sogenannte Helmholtz-Doppelschichten ausbilden, wie dies generell an jedem Phasenübergang zwischen einer metallischen Elektrode und einer elektrolytischen Flüssigkeit geschieht. Ursache für diese Schicht ist die unterschiedliche Art des Ladungstransports in den genannten Materialien. Die Helmholtz-Doppelschicht stellt in erster Näherung eine elektrische Kapazität dar, die sich bei der Stimulation des Zellgewebes auflädt. Dieser Effekt wird in der Fachwelt unter anderem mit dem Begriff Elektrodenpolarisation bezeichnet. An der aufgeladenen Elektrodenkapazität liegt dann eine Spannung an, die im folgenden als Polarisationsspannung bezeichnet wird.

Überschreitet die Polarisationsspannung einen gewissen Schwellwert, treten unerwünschte Redoxreaktionen auf, die zu irreversiblen Gewebeschäden führen können. Um dies zu vermeiden, sind im Stand der Technik verschiedene Maßnahmen bekannt.

In der genannten US 4,628,933 wird vorgeschlagen, zwischen den Stimulationselektroden und der zumindest einen Gegenelektrode (dort als ground conductor bezeichnet) einen großen Widerstand anzuordnen, über den sich die Polarisationsspannung abbauen kann. Als weitere Lösung wird die Beschichtung der Elektroden mit Bariumtitanat oder Iridium vorgeschlagen, da diese Materialien nur eine geringe Polarisationsneigung besitzen sollen. Außerdem soll die elektrische Stimulation mit einem rechteckförmigen Wechselsignal erfolgen, dessen Mittelwert bei Null liegt, so daß die Phasengrenzkapazität stets wieder entladen wird.

In dem DE-Buch "Die Bedeutung der Phasengrenze zwischen alloplastischen Festkörpern und biologischen Geweben für die Elektrostimulation" von Armin Bolz, erschienen im Fachverlag Schiele und Schön, 1994, wird im Hinblick auf die genannte Problematik vorgeschlagen, die Stimulationselektrode und die Gegenelektrode mit Hilfe eines sogenannten Autoshort-Schalters zyklisch kurzzuschließen, um einen schnellen Ladungsabbau an der Phasengrenze zu erreichen (a.a.0. Seite 49).

Ein weiterer Ansatz zur Vermeidung unerwünschter Reaktionen besteht darin, die Kapazität des Phasenübergangs durch die Gestaltung der Elektrodenflächen möglichst groß zu bemessen, um so die Polarisationsspannung zu minimieren. Darüber hinaus können Parameter des Stimulussignals, wie insbesondere die Impulsdauern und -amplituden, so dimensioniert werden, daß unerwünschte Reaktionen selbst unter "worst case"- Bedingungen ausgeschlossen sind. Schließlich ist es grundsätzlich möglich, die Polarisationsspannung aus den Impulsdauern und Impulsamplituden des Stimulussignals zu berechnen, um die Stimulation auf der Basis der Ergebnisse zu steuern.

Keines der bekannten Verfahren ist jedoch optimal. Gerade die zuletzt genannten Maßnahmen besitzen den Nachteil, daß in erster Linie transiente Vorgänge erfaßt werden, eine im Laufe der Zeit ansteigende statische Elektrodenpolarisation jedoch außer Betracht bleibt. Daher müssen in der Praxis Sicherheitsreserven bei der Dimensionierung berücksichtigt werden, was eine optimale Stimulation des Zellgewebes erschwert.

Das regelmäßige oder kontinuierliche Entladen der Phasengrenzkapazität beeinträchtigt die Freiheitsgrade bei der zeitlichen Dimensionierung der Stimulussignale. Ebenso schränkt die Verwendung nullpunktsymmetrischer Wechselsignale die Gestaltungsfreiheit ein. Hinzu kommt, daß sich einige der Maßnahmen zwar gut eignen, um unerwünschte Reaktionen im Bereich einer oder weniger Elektroden auszuschließen. Wird für die Stimulation des Zellgewebes jedoch, wie bspw. bei einem Retina-Implantat, eine Vielzahl von Elektroden, d. h. ein Multielektrodenarray, verwendet, ist eine Echtzeit-Überwachung zur Vermeidung unerwünschter Reaktionen sehr aufwendig.

Aus der US 5,674,264 A und der US 6,301,505 B1 ist jeweils eine Vorrichtung zur elektrischen Stimulation von biologischem Material bekannt, in der eine eventuell auftretende Polarisationsspannung an einer Stimulationselektrode ermittelt und die Stimulationsstrategie dahingehend beeinflusst wird, einen Abbau der Spannung zu begünstigen. Diese Vorrichtungen sind Cochlear-Implantate mit einer entsprechenden Ausgestaltung des Elektrodenträgers.

Vor diesem Hintergrund liegen der vorliegenden Erfindung die Aufgabe zu Grunde, eine auftretende Polarisationsspannung an der Stimulationselektrode abzubauen bzw. zu vermeiden.

Bei dem eingangs genannten Retina-Implantat wird diese Aufgabe dadurch gelöst, daß zumindest eine Sensorelektrode vorhanden ist, mit der eine Polarisationsspannung an der Stimulationselektrode bestimmbar ist, und daß eine Vielzahl von Stimulationselektroden und eine Vielzahl von Sensorelektroden auf einem gemeinsamen Träger angeordnet sind.

Der hier vorgeschlagenen Lösung liegt im Gegensatz zu der Vielzahl der bisherigen Ansätze der Gedanke zugrunde, die tatsächliche Polarisation an der Stimulationselektrode möglichst exakt und in Echtzeit zu erfassen, um in Abhängigkeit der gewonnenen, realen Meßergebnisse eine optimale Steuerung des Stimulussignals zu ermöglichen. Dieser Ansatz erlaubt erstmals eine weitreichende Gestaltungsfreiheit bei der Dimensionierung des Stimulussignals, ohne daß die Gefahr unerwünschter Reaktionen im Bereich der Stimulationselektroden unkontrolliert ansteigt. Während alle bisherigen Ansätze bewußt oder unbewußt vermieden haben, weitere Phasengrenzen, die ja die Grundursache der unerwünschten Reaktionen sind, durch zusätzliche Elektroden zu schaffen, geht die vorliegende Erfindung gerade diesen Weg. Es hat sich nämlich überraschenderweise gezeigt, daß die neuen Gestaltungsmöglichkeiten bisher zu erwartende Nachteile sehr gut kompensieren.

Es hat sich insbesondere gezeigt, daß die an sich zu erwartenden Risiken infolge der zusätzlichen Phasengrenzflächen erheblich reduziert sind, wenn man den Stromfluß über die Grenzfläche reduziert oder sogar weitgehend unterdrückt. Gerade dies ist bei der erfindungsgemäßen Sensorelektrode sehr gut möglich, da die Bestimmung der Polarisationsspannung auch ohne nennenswerten Stromfluß realisierbar ist. Überraschenderweise kann die erfindungsgemäße Sensorelektrode daher ohne Erhöhung des Risikos unerwünschter Redoxreaktionen eingesetzt werden.

Darüber hinaus war bislang auch nicht zu erwarten, daß die Polarisationsspannung an der Stimulationselektrode überhaupt mit Hilfe einer zusätzlichen, im Gewebekontakt stehenden Sensorelektrode bestimmt werden kann.

Die erfindungsgemäße Lösung besitzt insbesondere im Zusammenhang mit Multielektrodenarrays den Vorteil, daß die Vermeidung unerwünschter Redoxreaktionen in Echtzeit möglich ist, so daß Schädigungen des Zellgewebes besonders zuverlässig ausgeschlossen werden können. Außerdem lassen sich dabei auch statische Anteile der Elektrodenpolarisation zuverlässig erfassen, wodurch die Sicherheit weiter erhöht ist.

Ein weiterer Vorteil besteht darin, daß das Stimulussignal bei chronischen Implantaten an langsame Veränderungen der Kontaktsituation angepaßt werden kann. Eine Veränderung der Kontaktsituation ergibt sich bspw., wenn sich im Bereich der Elektroden infolge der Stimulation fibrotisches Gewebe bildet.

Schließlich erlaubt die erfindungsgemäße Sensorelektrode auch unabhängig von der Vermeidung unerwünschter Reaktionen erstmals eine exakte Regelung des Stimulussignals, und zwar in Echtzeit und in Abhängigkeit der realen Umgebungsbedingungen. Hierdurch kann die Stimulation des Gewebes sehr gezielt und fein dosiert durchgeführt werden.

In einer Ausgestaltung der Erfindung beinhaltet die Elektrodenanordnung ferner ein Differenzglied mit zumindest zwei Eingängen, wobei ein erster Eingang mit der Stimulationselektrode und ein zweiter Eingang mit der Sensorelektrode verbunden ist.

Bevorzugt handelt es sich bei dem Differenzglied um einen Differenzverstärker in an sich bekannter Schaltungstechnik. Durch den hochohmigen Eingang solcher Differenzverstärker wird der Stromfluß bei der Bestimmung der Polarisationsspannung sehr stark reduziert, so daß eine zusätzliche, das Meßsignal verfälschende Polarisationsspannung an der Sensorelektrode vermieden wird und folglich die bereits zuvor genannten Vorteile besonders stark zur Geltung kommen. Die Maßnahme besitzt zudem den Vorteil, daß das Differenzglied die relevante Polarisationsspannung an der Stimulationselektrode unmittelbar und ohne weitere Rechenschritte bereitstellt. Infolge dessen ist eine Echtzeit-Überwachung der Elektrodenpolarisation auf sehr einfache und effektive Weise möglich.

In einer weiteren Ausgestaltung beinhaltet die Elektrodenanordnung ferner einen Unterbrecher, der in Abhängigkeit von der bestimmten Polarisationsspannung das Stimulussignal unterbricht.

Durch diese Maßnahme wird praktisch ein Not-Aus-Schalter realisiert, mit dem ein von außen angelegtes Stimulussignal sofort unterbrochen werden kann, wenn die Elektrodenpolarisation kritische Werte erreicht. Hierdurch lassen sich unerwünschte Redoxreaktionen noch zuverlässiger unterbinden.

In einer weiteren Ausgestaltung der zuvor genannten Maßnahme ist der Unterbrecher ein Umschalter, der die Stimulationselektrode und die Gegenelektrode kurzschließt.

Durch diese Maßnahme läßt sich die Elektrodenpolarisation bei Erreichen eines kritischen Schwellwertes besonders schnell abbauen, wodurch die Zuverlässigkeit der Anordnung nochmals gesteigert ist.

In einer weiteren Ausgestaltung beinhaltet die Elektrodenanordnung ferner einen Regelkreis, der in Abhängigkeit von der bestimmten Polarisationsspannung zumindest einen Parameter des Stimulussignals verändert.

Diese Maßnahme zielt weniger auf das abrupte Abschalten eines Stimulussignals zur Vermeidung unerwünschter Redoxreaktionen, als vielmehr auf eine optimale Einstellung des Stimulussignals. Insbesondere können Parameter, wie Impulsdauern oder Impulsamplituden, und sogar der Dynamikbereich des Stimulussignals selbst sehr exakt eingestellt werden. Die Maßnahme besitzt den Vorteil, daß sich die eigentliche Stimulation des Zellgewebes in einem optimalen Arbeitsbereich durchführen läßt, wobei gleichzeitig die Vermeidung von unerwünschten Redoxreaktionen gewährleistet ist. Das abrupte Abschalten eines Stimulussignals kann damit weitgehend vermieden werden.

In einer weiteren Ausgestaltung beeinhaltet die Elektrodenanordung ferner einen Speicher, in dem ein Maximalwert der Polarisationsspannung abspeicherbar ist. Bevorzugt ist dieser Speicher drahtlos beschreibbar und/oder auslesbar.

Die Maßnahme besitzt den Vorteil, daß ein maximal zulässiger Höchstwert der Polarisationsspannung auf einfache Weise hinterlegt werden kann, so daß sich die Elektrodenanordnung einfach an individuelle Bedürfnisse eines Patienten anpassen läßt. Der abgespeicherte Maximalwert kann insbesondere als Vergleichsschwelle herangezogen werden, bei deren Überschreiten der zuvor genannte Unterbrecher eine Notabschaltung des Stimulussignals vornimmt. Alternativ oder ergänzend kann der Speicher auch im Betrieb der Elektrodenanordnung mit dem jeweils höchsten gemessenen Wert der Polarisationsspannung beschrieben werden. Diese Maßnahme ermöglicht eine spätere Kontrolle und Dokumentation einer durchgeführten Stimulierung. Die drahtlose Anbindung des Speichers ist besonders bevorzugt, wenn die Elektrodenanordnung, bspw. im Rahmen eines Retina-Implantats, fest im menschlichen Körper implantiert ist. In diesem Fall ist ein einfacher Zugriff auf den Speicher von außen möglich.

In einer weiteren Ausgestaltung ist die Sensorelektrode in unmittelbarer Nähe der Stimulationselektrode angeordnet.

Durch diese Maßnahme wird eine besonders exakte Bestimmung der kritischen Polarisation der Stimulationselektrode ermöglicht. Infolge dessen kann das Stimulussignal mit einer sehr engen Toleranz in einem optimalen Bereich geführt werden.

In einer weiteren Ausgestaltung ist die Sensorelektrode neben der Stimulationselektrode angeordnet.

Diese Maßnahme ist in produktionstechnischer Hinsicht sehr einfach und ermöglicht damit eine kostengünstige Realisierung der erfindungsgemäßen Anordnung.

In einer weiteren Ausgestaltung ist die Sensorelektrode konzentrisch zu der Stimulationselektrode angeordnet.

Diese Maßnahme ist besonders vorteilhaft, um die Polarisation der Stimulationselektrode mit der Sensorelektrode möglichst exakt zu erfassen, ohne Kompromisse hinsichtlich der effektiv wirksamen Fläche der Stimulationselektrode einzugehen. Wie bereits weiter oben angedeutet, hat eine große Phasengrenzkapazität den Vorteil, daß die Elektrodenpolarisation minimiert wird. Eine große Phasengrenzkapazität läßt sich wiederum durch eine möglichst große Fläche der Stimulationselektrode erreichen. Die konzentrische Anordnung ermöglicht eine optimale Verbindung der Forderungen nach großer Fläche der Stimulationselektrode und räumlich dichter, aber nicht einschränkender Anordnung der Sensorelektrode.

In einer weiteren Ausgestaltung umgibt die Sensorelektrode die Stimulationselektrode zumindest teilweise.

Besonders bevorzugt ist es, wenn die Sensorelektrode als dünner Ring um die Stimulationselektrode herum angeordnet ist. Diese Maßnahme ist eine besonders effiziente Möglichkeit, um die Elektrodenanordnung im Hinblick auf die zuvor genannten Parameter zu optimieren.

In einer alternativen Ausgestaltung ist die Sensorelektrode in einer Aussparung der Stimulationselektrode angeordnet.

Auch diese Maßnahme ist sehr gut geeignet im Hinblick auf eine optimale Anordnung der Sensorelektrode bei gleichzeitiger Optimierung der Stimulationselektrode.

In einer weiteren Ausgestaltung sind die Sensorelektrode und die Stimulationselektrode planare Strukturen.

Die Maßnahme ist besonders vorteilhaft im Hinblick auf Multielektrodenarrays, da planare Strukturen eine kostengünstige Herstellung einer Vielzahl von Elektroden ermöglichen. Dabei lassen sich insbesondere die zuvor genannten vorteilhaften Ausgestaltungen besonders einfach und kostengünstig realisieren.

In einer weiteren Ausgestaltung ist die Sensorelektrode klein im Vergleich zu der Stimulationselektrode.

Dabei bezieht sich die Größenangabe hier vor allem auf die wirksame Fläche der Sensorelektrode bzw. der Stimulationselektrode. Je kleiner die Sensorelektrode ist, desto mehr Platz steht für die Gestaltung der Stimulationselektrode zur Verfügung, wodurch sich die bereits erwähnte, vorteilhaft große Kapazität realisieren läßt. Darüber hinaus wird die Stimulation des Zellgewebes bei einer kleinen Sensorelektrode nicht nachteilig beeinflußt.

In einer weiteren Ausgestaltung sind die Sensorelektrode und die Stimulationselektrode aus gleichem Material hergestellt.

Diese Maßnahme ist besonders vorteilhaft im Hinblick auf die Herstellungskosten, da die beiden Elektroden hierdurch in einem gemeinsamen Produktionsschritt hergestellt werden können. Darüber hinaus werden elektrochemische Spannungen zwischen den beiden Elektroden vermieden, was ansonsten sowohl die Meßsignale als auch die Stimulussignale nachteilig beeinflussen könnte.

In einer weiteren Ausgestaltung der zuvor genannten Maßnahme ist im Bereich jeder Stimulationselektrode zumindest eine Sensorelektrode angeordnet.

Durch diese Maßnahme wird eine besonders exakte Regelung der Stimulussignale ermöglicht. Die Maßnahme ist besonders vorteilhaft bei Multielektrodenarrays, und darüber hinaus in Kombination mit den zuvor beschriebenen geometrischen Elektrodenformen.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
Fig. 1 eine Schaltungsskizze einer bevorzugten erfindungsgemä-ßen Elektrodenanordnung,
Figs. 2 bis 4 bevorzugte Ausführungsbeispiele von Stimulations- und Sensorelektroden, und
Fig. 5 eine vereinfachte Darstellung eines Multielektrodenarrays in planarer CMOS-Technologie mit der erfindungsgemäßen Elektrodenanordnung aus Fig. 1.

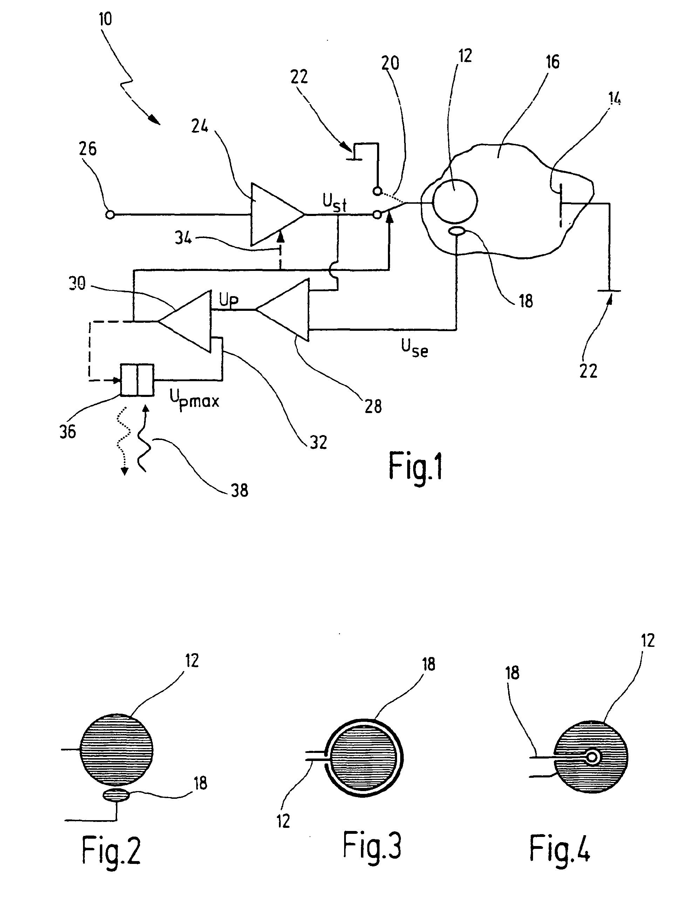

In Fig. 1 ist eine erfindungsgemäße Elektrodenanordnung in ihrer Gesamtheit mit der Bezugsziffer 10 bezeichnet.

Die Elektrodenanordnung 10 beinhaltet eine Stimulationselektrode 12, die in planarer Technologie hergestellt ist. Die Erfindung ist jedoch nicht hierauf beschränkt. In anderen Ausführungsbeispielen sind die Stimulations- und die Sensorelektrode in einer "3D-Anordnung", d.h. in zueinander versetzten Ebenen angeordnet. Mit der Bezugsziffer 14 ist eine Gegenelektrode bezeichnet, die den Gegenpol (Masse) für die Stimulationselektrode 12 bildet. Die beiden Elektroden 12, 14 dienen dazu, biologisches Material, im vorliegenden Beispiel ein Zellgewebe 16, in an sich bekannter Weise elektrisch zu stimulieren.

In dem gezeigten Ausführungsbeispiel ist das Zellgewebe 16 ein Gewebe im Bereich der Retina des menschlichen Auges. Die Stimulationselektrode 12 sowie die weiteren nachfolgend beschriebenen Bestandteile der Elektrodenanordnung 10 sind dabei entweder epiretinal oder bevorzugt subretinal in das Auge des Patienten implantiert.

Mit der Bezugsziffer 18 ist entsprechend der vorliegenden Erfindung eine Sensorelektrode bezeichnet, die in unmittelbarer Nähe, jedoch unabhängig von der Stimulationselektrode angeordnet ist. Die Fläche der Sensorelektrode 18 ist klein im Vergleich zu der Fläche der Stimulationselektrode 12.

Mit der Bezugsziffer 20 ist ein Umschalter bezeichnet, der die Stimulationselektrode 12 wahlweise mit einem von zwei alternativen Potentialen verbindet. Mit der Bezugsziffer 22 ist ein Massepotential bezeichnet, das mit dem festen Potential der Gegenelektrode identisch ist. Der Umschalter 20 verbindet die Stimulationselektrode 12 mit dem Massepotential 22, wenn mit Hilfe der Sensorelektrode 18 eine kritische Polarisation der Stimulationselektrode detektiert wurde. Auf diese Weise kann die kritische Polarisation der Stimulationselektrode 12 sehr schnell abgebaut werden, um unerwünschte Redoxreaktionen im Bereich des Zellgewebes 16 zu vermeiden.

In der alternativen zweiten Schaltstellung verbindet der Umschalter 20 die Stimulationselektrode 12 mit dem Ausgang eines Signalverstärkers 24, an dessen Eingang 26 ein Stimulussignal angelegt werden kann. Diese Schaltstellung des Umschalters 20 ist der Normalfall im bestimmungsgemäßen Betrieb der Elektrodenanordnung 10.

Entsprechend dem Ausführungsbeispiel, bei dem die Elektrodenanordnung 10 Bestandteil des erfindungsgemäßen Retina-Implantats ist, wird das Stimulussignal mit Hilfe von lichtempfindlichen Elementen (Multi-Photodioden-Array, hier nicht dargestellt) erzeugt. Der grundsätzliche Aufbau eines solchen Retina-Implantats ist bspw. aus DE 199 21 399 A1, DE 197 05 988 A1 oder aus DE 195 29 371 A1 bekannt, auf die hier hinsichtlich weiterer Details des Retina-Implantats in vollem Umfang Bezug genommen wird.

Mit der Bezugsziffer 28 ist ein Differenzverstärker bezeichnet, an dessen erstem Eingang die mit der Sensorelektrode 18 aufgenommene Spannung Uₛₑ anliegt. Am zweiten Eingang liegt das Ausgangssignal des Signalverstärkers 24 an, das im normalen Arbeitsbetrieb der Elektrodenanordnung 10 der Betriebsspannung Uₛₜ der Stimulationselektrode 12 entspricht. Der Differenzverstärker 28 bildet die Differenz der beiden Spannungen Uₛₜ und Uₛₑ, die der relevanten Polarisationsspannung Up entspricht (Uₚ = Uₛₜ - Uₛₑ). Das Ausgangssignal des Differenzverstärkers 28, d. h. die bestimmte Polarisationsspannung Uₚ ist einem ersten Eingang eines Komparators 30 zugeführt. An dessen zweitem Eingang 32 liegt eine Vergleichsspannung an, die in dem dargestellten bevorzugten Ausführungsbeispiel die maximal zulässige Polarisationsspannung Uₚₘₐₓ darstellt. Das Ausgangssignal des Komparators 30 bestimmt die Schaltstellung des Umschalters 20. Darüber hinaus bestimmt es in einem bevorzugten Ausführungsbeispiel der Erfindung auch den Verstärkungsfaktor des Signalverstärkers 24, dessen Dynamikbereich oder einen anderen geeigneten Parameter. Diese Abhängigkeit ist in Fig. 1 durch einen Signalabgriff 34 dargestellt. Es ergibt sich auf diese Weise ein Regelkreis für die optimale Einstellung des Stimulussignals.

Abweichend von dem in Fig. 1 gezeigten Ausführungsbeispiel kann der Signalabgriff 34 auch direkt am Ausgang des Differenzverstärkers 28 abgenommen sein. In diesem Fall ist das Stellsignal für den Signalverstärker 24 unmittelbar proportional zu der bestimmten Polarisationsspannung, d.h. es ist dann nicht über den Komparator "digitalisiert".

Mit der Bezugsziffer 36 ist ein Speicher bezeichnet, der hier drahtlos von außen beschreibbar und auslesbar ist. Dies ist durch die Pfeile 38 angedeutet. In dem Speicher 36 ist hier der maximal zulässige Höchstwert der Polarisationsspannung Uₚₘₐₓ abgespeichert. Des weiteren können die jeweils gemessenen Höchstwerte der Polarisationsspannung Uₚ oder, wie in Fig. 1 gezeigt, das Ausgangssignal des Komparators 30 in dem Speicher 36 abgespeichert werden, um eine spätere Kontrolle und Dokumentation der Stimulierung zu ermöglichen.

Die Elektrodenanordnung 10 ermöglicht verschiedene Steuerungen oder Regelungen des Stimulussignals. Beispielsweise ist es möglich, das Zellgewebe 16 mit Stromimpulsen zu stimulieren, wobei die an der Stimulationselektrode 12 anliegende Spannung ensprechend der bekannten Ladekurve einer Kapazität ansteigt. Mit Hilfe der Sensorelektrode 18 und des Differenzverstärkers 28 kann der Stromimpuls abgeschaltet werden, wenn die Polarisation der Stimulationselektrode 12 den maximal zulässigen Wert Uₚₘₐₓ überschreitet. In diesem Fall beinhaltet die Elektrodenanordnung 10 gewissermaßen eine Sicherheitsschaltung.

In einer anderen Anwendung dient die Elektrodenanordnung 10 eher als Adaptionsschaltung zum Einstellen eines optimalen Stimulussignals. Durch die Bestimmung der jeweils aktuellen Polarisationsspannung Up mit Hilfe der Sensorelektrode 18 können die maximal zulässigen Parameter (Amplitude, Impulsdauer, etc.) des Stimulussignals ermittelt und eingestellt werden. In manchen Anwendungen ist dabei das Ausgangssignal des Differenzverstärkers 28 über einen hier nicht gesondert dargestellten Abgriff auch nach außen geführt, um dort als Meßwert auch für externe Regel- und Meßkreise zur Verfügung zu stehen.

Bei der Anwendung der Elektrodenanordnung 10 bei einem erfindungsgemäßen Retina-Implantat wird das Ausgangssignal des Differenzverstärkers 28 vorteilhaft auch dazu verwendet, den. Arbeitspunkt und damit die Zaichtempfindlichkeit der lichtempfindlichen Elemente einzustellen. Bei einem Retina-Implantat gemäß der bereits erwähnten DE 199 21 399 A1 kann auf diese Weise vorteilhaft auch der Arbeitspunkt der dort beschriebenen Referenzelemente eingestellt werden.

Die Elektrodenanordnung 10 ist bevorzugt in CMOS-Technologie realisiert, was aufgrund der verwendeten analogen Bauelemente sehr einfach und kostengünstig möglich ist.

In den Figuren 2 bis 4 sind bevorzugte Ausführungsbeispiele von Stimulationselektroden 12 und Sensorelektroden 18 dargestellt. Die verwendeten Bezugszeichen entsprechen dabei denen aus Fig. 1. In Fig. 2 ist die Sensorelektrode 18 ein im Vergleich zur Stimulationselektrode 12 kleiner "Patch", der neben der Stimulationselektrode 12 angeordnet ist. In Fig. 3 umgibt die Sensorelektrode 18 die Stimulationselektrode 12 konzentrisch in Form eines dünnen Kreisrings. In Fig. 4 ist die Stimulationselektrode 18 mittig in einer Aussparung im Zentrum der Stimulationselektrode 12 angeordnet. In diesem Fall umgibt die Stimulationselektrode 12 die Sensorelektrode 18 konzentrisch.

In allen drei Fällen ist die Stimulationselektrode 12 jeweils kreisförmig dargestellt. Eine solche Ausführung hat sich in der Praxis als vorteilhaft erwiesen. Darüber hinaus sind jedoch auch andere Geometrien, bspw. quadratische Elektrodenflächen möglich. Die Sensorelektrode 18 besitzt dabei eine entsprechend angepaßte Geometrie.

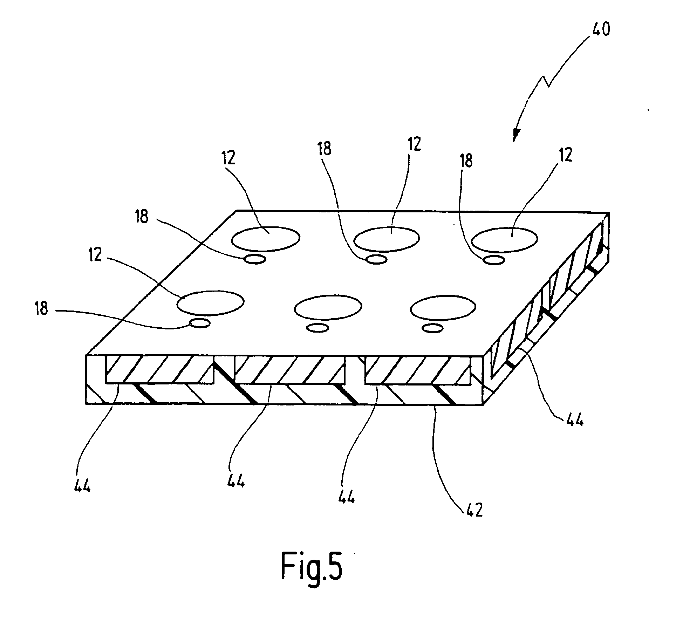

In Fig. 5 ist ein Multielektrodenarray in seiner Gesamtheit mit der Bezugsziffer 40 bezeichnet. Das Multielektrodenarray, das Bestandteil eines Retina-Implantats ist, beinhaltet einen Träger 42, auf dem eine Vielzahl von Stimulationselektroden 12 und Sensorelektroden 18 angeordnet sind. Des weiteren sind in den Träger 42 in an sich bekannter Weise CMOS-Strukturen 44 eingebettet, mit denen der Umschalter 20, der Signalverstärker 24, der Differenzverstärker 28, der Komparator 30 und weitere hier nicht näher dargestellte Schaltungsbestandteile realisiert sind. Bei dem Retina-Implantat enthält der Träger 42 (Chip) ferner auch die lichtempfindlichen Elemente sowie die weitere benötigte Steuerelektronik.

Die Vorteile der erfindungsgemäßen Elektrodenanordnung kommen dabei um so mehr zur Geltung, je höher die Anzahl und damit die Packungsdichte der Stimulationselektroden ist.

## Patentansprüche

1. Retina-Implantat mit einer Elektrodenanordnung zur elektrischen Stimulation von biologischem Material (16) im Bereich der Retina eines Auges, wobei die Elektrodenanordnung mit zumindest einer Stimulationselektrode (12), über die dem biologischen Material (16) ein Stimulussignal zuführbar ist, und mit zumindest einer Gegenelektrode (14) versehen ist, die einen Gegenpol zu der Stimulationselektrode- (12) bildet, **dadurch gekennzeichnet, daß** ferner zumindest eine Sensorelektrode (18) vorhanden ist, mit der eine Polarisationsspannung an der Stimulationselektrode (12) bestimmbar ist, und daß eine Vielzahl von Stimulationselektroden (12) und eine Vielzahl von Sensorelektroden (18) auf einem gemeinsamen Träger (42) angeordnet sind.

2. Retina-Implantat nach Anspruch 1, **dadurch gekennzeichnet, daß** sie ferner ein Differenzglied (28) mit zumindest zwei Eingängen beinhaltet, wobei ein erster Eingang mit der Stimulationselektrode (12) und ein zweiter Eingang mit der Sensorelektrode (18) verbunden ist.

3. Retina-Implantat nach Anspruch 1 oder 2, **dadurch gekennzeichnet, daß** sie ferner einen Unterbrecher (20) beinhaltet, der in Abhängigkeit von der bestimmten Polarisationsspannung das Stimulussignal unterbricht.

4. Retina-Implantat nach Anspruch 3, **dadurch gekennzeichnet, daß** der Unterbrecher (20) ein Umschalter ist, der die Stimulationselektrode (12) und die Gegenelektrode (14) kurzschließt.

5. Retina-Implantat nach einem der Ansprüche 1 bis 4, **dadurch gekennzeichnet, daß** sie ferner einen Regelkreis (28, 30, 34) beinhaltet, der in Abhängigkeit von der bestimmten Polarisationsspannung zumindest einen Parameter des Stimulussignals verändert.

6. Retina-Implantat nach einem der Ansprüche 1 bis 5, **dadurch gekennzeichnet, daß** sie ferner einen Speicher (36) beinhaltet, in dem ein Maximalwert der Polarisationsspannung abspeicherbar ist.

7. Retina-Implantat nach Anspruch 6, **dadurch gekennzeichnet, daß** der Speicher (36) drahtlos (38) beschreibbar und/oder auslesbar ist.

8. Retina-Implantat nach einem der Ansprüche 1 bis 7, **dadurch gekennzeichnet, daß** die Sensorelektrode (18) in unmittelbarer Nähe der Stimulationselektrode (12) angeordnet ist.

9. Retina-Implantat nach einem der Ansprüche 1 bis 8, **dadurch gekennzeichnet, daß** die Sensorelektrode (18) neben der Stimulationselektrode (12) angeordnet ist.

10. Retina-Implantat nach einem der Ansprüche 1 bis 9, **dadurch gekennzeichnet, daß** die Sensorelektrode (18) konzentrisch zu der Stimulationselektrode (12) angeordnet ist.

11. Retina-Implantat nach einem der Ansprüche 1 bis 10, **dadurch gekennzeichnet, daß** die Sensorelektrode (18) die Stimulationselektrode (12) zumindest teilweise umgibt.

12. Retina-Implantat nach einem der Ansprüche 1 bis 10, **dadurch gekennzeichnet, daß** die Sensorelektrode (18) in einer Aussparung der Stimulationselektrode (12) angeordnet ist.

13. Retina-Implantat nach einem der Ansprüche 1 bis 12, **dadurch gekennzeichnet, daß** die Sensorelektrode (18) und die Stimulationselektrode (12) planare Strukturen sind.

14. Retina-Implantat nach einem der Ansprüche 1 bis 13, **dadurch gekennzeichnet, daß** die Sensorelektrode (18) klein im Vergleich zu der Stimulationselektrode (12) ist.

15. Retina-Implantat nach einem der Ansprüche 1 bis 14, **dadurch gekennzeichnet, daß** die Sensorelektrode und die Stimulationselektrode aus gleichem Material hergestellt sind.

16. Retina-Implantat nach einem der Ansprüche 1 bis 15, **dadurch gekennzeichnet, daß** im Bereich jeder Stimulationselektrode (12) zumindest eine Sensorelektrode (18) angeordnet ist.

## Claims

1. A retina implant having an electrode arrangement for electrical stimulation of biological material (16) in the area of the retina of a human eye, wherein said electrode arrangement has at least one stimulation electrode (12) via which the biological material (16) can be fed with a stimulus signal, and with at least one counter electrode (14) which forms a counter pole to said stimulation electrode (12), **characterized in that** further at least one sensor electrode (18) is provided with the aid of which it is possible to determine a polarization voltage at the stimulation electrode (12), and that a multiplicity of stimulation electrodes (12) and a multiplicity of sensor electrodes (18) are arranged on a common substrate (42).

2. The retina implant of claim 1, **characterized in that** it further includes a differential element (28) having at least two inputs, a first input being connected to the stimulation electrode (12) and a second input being connected to the sensor electrode (18).

3. The retina implant of claim 1 or claim 2, **characterized in that** it further includes an interrupter (20) which interrupts the stimulus signal as a function of the determined polarization voltage.

4. The retina implant of claim 3, **characterized in that** said interrupter (20) is a change over switch which short-circuits the stimulation electrode (12) and the counter electrode (14).

5. The retina implant of anyone of claims 1 through 4, **characterized in that** it further comprises a control loop (28, 30, 34) which varies at least one parameter of the stimulation signal as a function of the determined polarization voltage.

6. The retina implant of anyone of claims 1 through 5, **characterized in that** it further comprises a memory (36) in which a maximum value of the polarization voltage can be stored.

7. The retina implant of claim 6, **characterized in that** said memory (36) can be written to and/or read from in a wireless fashion.

8. The retina implant of anyone of claims 1 through 7, **characterized in that** the sensor electrode (18) is arranged in immediate vicinity of the stimulation electrode (12).

9. The retina implant of anyone of claims 1 through 8, **characterized in that** the sensor electrode (18) is arranged next to the stimulation electrode (12).

10. The retina implant of anyone of claims 1 through 9, **characterized in that** the sensor electrode (18) is arranged concentrically with the stimulation electrode (12).

11. The retina implant of anyone of claims 1 through 10, **characterized in that** the sensor electrode (18) at least partially surrounds the stimulation electrode (12).

12. The retina implant of anyone of claims 1 through 10, **characterized in that** the sensor electrode (18) is arranged in a cut-out of the stimulation electrode (12).

13. The retina implant of anyone of claims 1 through 12, **characterized in that** the sensor electrode (18) and the stimulation electrode (12) are planar structures.

14. The retina implant of anyone of claims 1 through 13, **characterized in that** the sensor electrode (18) is small as compared to the stimulation electrode (12).

15. The retina implant of anyone of claims 1 through 14, **characterized in that** the sensor electrode and the stimulation electrode are produced from the same material.

16. The retina implant of anyone of claims 1 through 15, **characterized in that** at least one sensor electrode (18) is arranged in the region of each stimulation electrode (12).

## Revendications

1. Implant de rétine avec un agencement d'électrodes pour la stimulation électrique de matériau (16) biologique dans la zone de la rétine d'un oeil humain, l'agencement d'électrodes étant doté d'au moins une électrode de stimulation (12), par laquelle un signal stimulus peut être amené au matériau (16) biologique, et d'au moins une contre-électrode (14), qui forme un contre-pôle à l'électrode de stimulation (12), **caractérisé en ce qu'**il est prévu de plus au moins une électrode de détection (18) avec laquelle une tension de polarisation peut être déterminée sur l'électrode de stimulation (12), et **en ce qu'**une pluralité d'électrodes de stimulation (12) et une pluralité d'électrodes de détection (18) sont disposées sur un support (42) commun.

2. Implant de rétine selon la revendication 1, **caractérisé en ce qu'**il contient de plus un élément différentiel (28) avec au moins deux entrées, une première entrée étant reliée à l'électrode de stimulation (12) et une seconde entrée à l'électrode de détection (18).

3. Implant de rétine selon la revendication 1 ou 2, **caractérisé en ce qu'**il contient de plus un dispositif d'interruption (20) qui interrompt le signal stimulus en fonction de la tension de polarisation déterminée.

4. Implant de rétine selon la revendication 3, **caractérisé en ce que** le dispositif d'interruption (20) est un inverseur qui court-circuite l'électrode de stimulation (12) et la contre-électrode (14).

5. Implant de rétine selon l'une quelconque des revendications 1 à 4, **caractérisé en ce qu'**il contient également un circuit régulateur (28, 30, 34), qui modifie au moins un paramètre du signal stimulus en fonction de la tension de polarisation déterminée.

6. Implant de rétine selon l'une quelconque des revendications 1 à 5, **caractérisé en ce qu'**il contient également une mémoire (36) dans laquelle une valeur maximale de la tension de polarisation peut être mémorisée.

7. Implant de rétine selon la revendication 6, **caractérisé en ce que** la mémoire (36) peut être décrite et/ou lue sans fil (38).

8. Implant de rétine selon l'une quelconque des revendications 1 à 7, **caractérisé en ce que** l'électrode de détection (18) est disposée à proximité immédiate de l'électrode de stimulation (12).

9. Implant de rétine selon l'une quelconque des revendications 1 à 8, **caractérisé en ce que** l'électrode de détection (18) est disposée à côté de l'électrode de stimulation (12).

10. Implant de rétine selon l'une quelconque des revendications 1 à 9, **caractérisé en ce que** l'électrode de détection (18) est disposée de façon concentrique par rapport à l'électrode de stimulation (12).

11. Implant de rétine selon l'une quelconque des revendications 1 à 10, **caractérisé en ce que** l'électrode de détection (18) entoure au moins partiellement l'électrode de stimulation (12).

12. Implant de rétine selon l'une quelconque des revendications 1 à 10, **caractérisé en ce que** l'électrode de détection (18) est disposée dans un évidement de l'électrode de stimulation (12).

13. Implant de rétine selon l'une quelconque des revendications 1 à 12, **caractérisé en ce que** l'électrode de détection (18) et l'électrode de stimulation (12) sont des structures planaires.

14. Implant de rétine selon l'une quelconque des revendications 1 à 13, **caractérisé en ce que** l'électrode de détection (18) est petite par rapport à l'électrode de stimulation (12).

15. Implant de rétine selon l'une quelconque des revendications 1 à 14, **caractérisé en ce que** l'électrode de détection et l'électrode de stimulation sont fabriquées dans le même matériau.

16. Implant de rétine selon l'une quelconque des revendications 1 à 15, **caractérisé en ce que** au moins une électrode de détection (18) est disposée dans la zone de chaque électrode de stimulation (12).
